(19) Europäisches Patentamt
European Patent Office
Office européen des brevets

(11) **EP 4 454 569 A1**

(12) **EUROPEAN PATENT APPLICATION**
published in accordance with Art. 153(4) EPC

(43) Date of publication:
**30.10.2024 Bulletin 2024/44**

(21) Application number: **21969149.0**

(22) Date of filing: **24.12.2021**

(51) International Patent Classification (IPC):
***A61B 8/14*** *(2006.01)*        ***A61B 8/08*** *(2006.01)*

(52) Cooperative Patent Classification (CPC):
**A61B 8/4466; A61B 8/08; A61B 8/14;
A61B 8/4461;** A61B 8/4483

(86) International application number:
**PCT/KR2021/019851**

(87) International publication number:
**WO 2023/120785 (29.06.2023 Gazette 2023/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Mcube Technology Co., Ltd.
Seoul 07789 (KR)**

(72) Inventor: **KIM, Jung Hoe
Seoul 06798 (KR)**

(74) Representative: **Engelhard, Markus
Boehmert & Boehmert
Anwaltspartnerschaft mbB
Pettenkoferstrasse 22
80336 München (DE)**

(54) **ULTRASONIC SCANNER, AND ULTRASONIC SIGNAL CORRECTION METHOD FOR ULTRASONIC SCANNER**

(57)    The present invention relates to an ultrasound scanner. The ultrasound scanner is a bladder scanner that uses mechanical rotation using a motor in one direction and manual rotation by an operator in the other direction. The ultrasound scanner is configured to reduce measurement errors in the volume of urine that may be caused by unwanted physical position movement of an ultrasound transducer composed of a single element when calculating the volume of urine in the bladder. The control unit of the ultrasound scanner according to the present invention is characterized by receiving ultrasound echo signals from the ultrasound transducer, correcting the coordinates of the ultrasound echo signals by considering the tilt angle of the ultrasound probe by manual operation, and obtaining a sector-shaped two-dimensional ultrasound image using the corrected coordinates of the ultrasound echo signals.

【 FIG. 12A】

**(Cont. next page)**

【 FIG. 12B】

95

| coordinate correction module | 950 |

| 2D ultrasound image acquisition module | 952 |

| 3D information extraction module | 954 |

## Description

[TECHNICAL FIELD]

The present invention relates to a portable bladder scanner that measures the volume of urine filling the bladder using a 3-dimensional scanning ultrasound scanner. More specifically, the present invention relates to an ultrasound scanner that extracts the physical position error of the ultrasound transducer generated during the ultrasound scanning process according to the manual rotation by the operator, corrects the coordinates of the ultrasound echo signal using the extracted physical position error and allows accurate measurement of the volume of urine inside the bladder.

[BACKGROUND ART]

Normal people can feel bladder fullness and urinate voluntarily when the bladder is filled with a certain amount of urine. However, patients with urinary disorders caused by various diseases do not feel their bladder full or cannot urinate voluntarily even if they feel their bladder full. This can cause too much urine to fill the bladder, and when this happens, there is a risk of developing bladder-related complications. In order to care for the patients with these urinary disorders, the volume of urine accumulated in the bladder must be measured periodically or whenever necessary situations arise. In this way, to measure the volume of the patient's urine, a hand-held, easily portable 3D ultrasound scanner is used.

In this specification, a special purpose portable 3D ultrasound scanner for measuring the volume of urine filled in the bladder is referred to as a 'bladder scanner'. Unlike general medical ultrasound scanners, the main purpose of the bladder scanner is to measure the volume of urine inside the bladder. Therefore, the main function of the bladder scanner is to automatically segment the bladder area from a 3D ultrasound image, calculate the volume of the segmented bladder area, and display the calculated volume of the bladder area as a numerical value. And, a secondary function of the bladder scanner is to output the acquired ultrasound image to a display device. In particular, the bladder scanner described above is characterized by minimizing its size and weight so that it can be carried by medical person. For this reason, the bladder scanner is specialized for measuring the volume of urine, and other secondary functions are generally eliminated.

In order to reduce the manufacturing cost of the bladder scanner, the bladder scanner is sometimes manufactured with a structure that includes an ultrasound transducer composed of a single element and motors that drive it. The bladder scanner with this structure often uses an operation method of scanning ultrasound beams while rotating the ultrasonic transducer using the motors for sector scans. In order to perform 3D ultrasound scanning of the bladder, it is necessary to sequentially perform a sector scan of a single plane in multiple directions to obtain multiple 2D cross-sectional ultrasound images of the bladder. This requires scanning the transducer in two directions. Additionally, for scanning in two directions, two motors driven in different directions can be provided. However, the ultrasound scanner with this structure has a problem in that the size and weight of the motor drive unit increase.

Therefore, in order to make the portable bladder scanner as small and light as possible, it can be manufactured with a structure equipped with a single motor. The bladder scanner of this structure is configured to automatically rotate in one direction using the single motor and to rotate manually in the other direction using the operator's hand movements. The present invention relates to a method of reducing measurement errors in volume of urine that may occur in the bladder scanner of the ultrasound scanning method using manual rotation by an operator.

Bladder scanners are manufactured to be used solely to measure the volume of the bladder, and therefore have the characteristics of being smaller in size and cheaper than general ultrasound scanners. To reduce the manufacturing cost of the bladder scanner, an ultrasound transducer consisting of a single element is generally equipped. The general ultrasound scanners are equipped with a phased array transducer in which multiple transducers are arranged in a line or plane shape, so they are expensive and the operation method is complicated. Therefore, the general ultrasound scanners have limitations that make it difficult to use them as portable bladder scanners. Therefore, in the bladder scanner, it is common to use an ultrasound transducer composed of a single element. The bladder scanner that uses an ultrasound transducer composed of a single element requires three-dimensional ultrasound scanning to obtain volumetric image of the bladder. The bladder scanner generates a 3-dimensional ultrasound image using a plurality of the 2-dimensional cross-section ultrasound images which are obtained by performing a plurality of the sector scans at various angles.

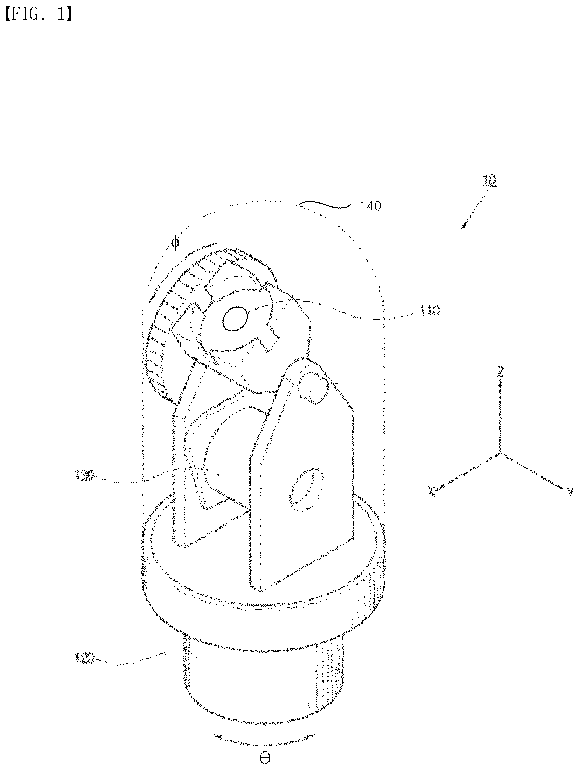

FIG. 1 is a structural diagram showing a general bladder scanner. Referring to FIG. 1, the general bladder scanner 10 includes a control unit that controls overall operation, an ultrasound transducer 110, a first motor 120, a second motor 130 and a motor drive unit. The bladder scanner described above has an ultrasound transducer 110 at the bottom. The thickness of the transducer is determined by the frequency of ultrasound, and the frequency used for bladder scanning is generally about 2-3 MHz. The Transducer is generally circular. The diameter of the transducer is determined by considering the maximum depth of the bladder to be measured by the ultrasound signal, and is generally about 10-15 mm. The ultrasound transducer 110 is mechanically connected to the motor drive unit. The motor drive unit performs ultrasound scanning by driving the first motor and the second motor to rotate the ultrasound transducer in two different directions. The motor drive unit generally uses a sector scan method to obtain a two-dimensional cross-sectional ultrasound image. The ultrasound transducer 110, the motor drive unit, and the ultrasound driving circuit unit are placed and fixed within the covering 140 of the bladder scanner.

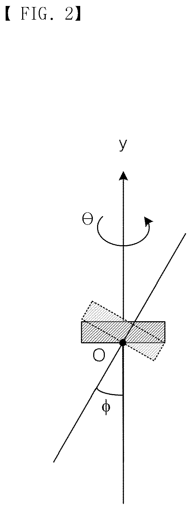

FIG. 2 is a schematic diagram illustrating a sector scan for obtaining two-dimensional ultrasound signals by rotating an ultrasound transducer using a motor in a general bladder scanner. As shown in FIG. 2, when the ultrasound transducer is rotated by an angle $\varphi$, the ultrasound beam coming from the transducer rotates by an angle $\varphi$ about the central axis y. At this time, the rotation axis of the ultrasound transducer places the center point 0 on the bottom surface of the ultrasound transducer.

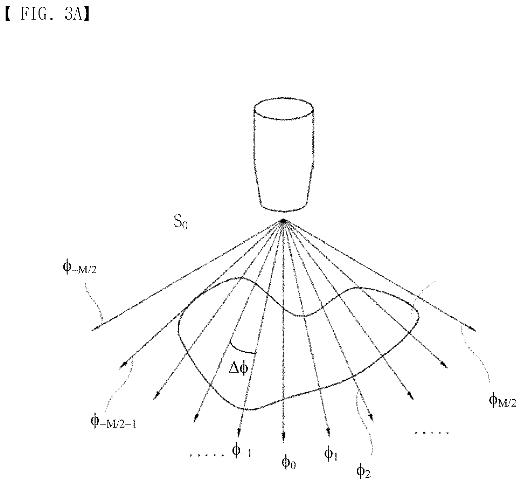

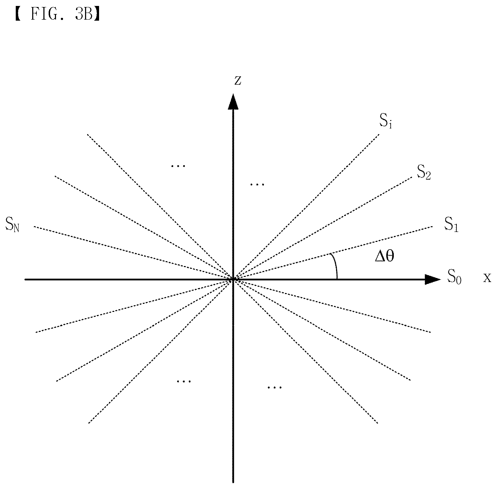

FIGS. 3A and 3B are diagrams showing the direction in which the rotation axis of the ultrasound transducer is rotated according to a conventional method in a general bladder scanner. When the motor drive unit rotates the transducer at a constant angular velocity, a sector scan as shown in FIG. 3A can be performed. In FIG. 3A, the number of scanning lines is M+1, and the separation angle $\Delta\varphi$ of adjacent scanning lines is preferably set to a constant value. The angle of the sector scan should be set to scan the entire bladder, and is usually around 120°. A sector scan generates one two-dimensional cross-sectional image. Therefore, in order to obtain a 3D image, the sector scans must be performed repeatedly while changing the scanning angle. A commonly used method is to perform the sector scans as shown in FIG. 3A while the transducer is rotated at a constant angular velocity about the central y-axis as shown in FIG. 3B. In FIG. 3B, this rotation angle is indicated as $\theta$. As shown in FIG. 3B, a three-dimensional scanning trajectory can be generated by performing one sector scan, rotating by $\Delta\theta$, and then repeating the sector scan. By sequentially rotating 180° along the $\theta$ direction, a 3D volume image can be obtained.

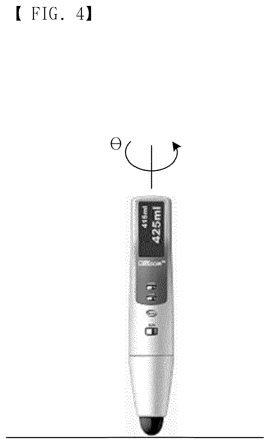

FIG. 4 is a schematic diagram showing the direction in which the bladder scanner is rotated by driving the motor to obtain a 3D image in a bladder scanner equipped with two motors driven in different directions. As shown in FIG. 4, by placing the bladder scanner on the patient's lower abdomen, that is, on the surface of the abdomen where the bladder is located, and then performing the three-dimensional scanning described above, a three-dimensional image of the area including the bladder can be obtained. The 3D scanning of a bladder scanner is very slow compared to a general ultrasound scanner, and the scanning speed is usually several seconds. The transducer reciprocates at a constant angular velocity within the sector plane ($S_i$ (i=0, 1, 2, ..., N)), transmits ultrasound pulses, and receives ultrasound echo signals reflected within the human body. If the number of sector planes is large, more precise three-dimensional scanning can be performed. However, if the number of sector planes is large, the measurement time of the bladder scanner becomes longer. Therefore, the number of sector planes is usually about 10 to 20.

In this way, in order to perform three-dimensional scanning using an ultrasound transducer composed of a single element, it is necessary to rotate the ultrasound transducer in two directions. Therefore, a scan structure with two degrees of freedom is needed. Typically, two motors are used to implement the scanning structure with two degrees of freedom, which causes the bladder scanner to become larger and heavier.

The sector scans are performed in a plurality of different directions including the bladder to obtain a plurality of two-dimensional ultrasound images for different positions, and the volume of urine can be measured by using the obtained plurality of two-dimensional ultrasound images. The algorithm for measuring the volume of urine from a plurality of two-dimensional ultrasound images is a known technology, and various methods are used. In particular, the urine volume measurement algorithms are described in detail in Korean Registration Patent No. 10-0763453, Korean Registration Patent No. 10-1874613, etc.

As the ultrasound pulse emitted from the transducer travels straight through the human body, its intensity gradually attenuates as it is partially reflected or scattered. The degree to which ultrasound pulses are reflected or scattered is determined by the difference between the acoustic impedances of the area and the surrounding area. Because muscle and internal tissue, which are common biological tissues, are made up of cells gathered together, reflection and scattering occur even within the same biological tissue. However, because urine in the bladder is a uniform liquid component, the uniformity of acoustic impedance is higher than that of the biological tissues. Therefore, reflection and scattering of ultrasound waves rarely occur within the urine. Therefore, the urine inside the bladder has low luminance in the ultrasound image. The bladder regions are segmented in 2-dimensional ultrasound images using the high contrast between the urine inside the bladder and the surrounding biological tissue, and a 3D image of the bladder can be obtained by aligning the segmented bladder regions in three dimensions. The Volume of urine can be measured from this 3D bladder image. As shown in FIG. 3A, when ultrasound scanning is performed, all scanning lines in all cross sections start from one-point 0, and therefore cross-sectional images obtained at all angles have the same magnification. In other words, a cone-shaped coordinate system with the point 0 as the starting point can be applied to each cross-sectional image, and as a result, the calculation of the volume of urine is simplified.

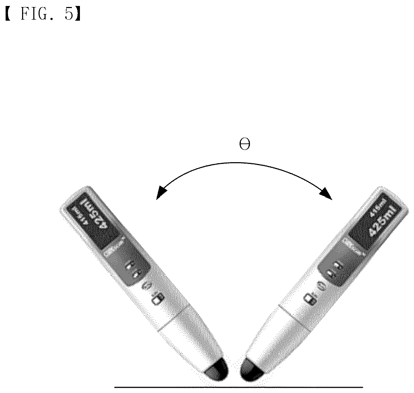

As described above, in order to make the bladder scanner smaller and lighter, it can be equipped with only a single motor. In a bladder scanner with a single motor, rotation in the first direction is achieved by driving the motor, and rotation in the second direction is achieved by manual movement of the operator. FIG. 5 is a schematic diagram showing rotation for acquiring a 3D image in a conventional bladder scanner with a single motor.

As shown in FIG. 5, while the operator manually tilts a bladder scanner along a second direction orthogonal to a first direction, the bladder scanner with a single motor uses the motor to automatically rotate the transducer along the first direction in order to perform a sector scan. The motor can produce angular motion at constant speed through electrical control. However, it is difficult to generate constant angular motion when the operator manually rotates the bladder scanner. Therefore, it is necessary to measure the angle of the scanner by attaching an angle sensor to the bladder scanner.

When the bladder scanner is placed on the surface of a person's abdomen and an ultrasound scan is performed by manual rotation by the operator, a problem occurs in which the starting points of the scanning beams do not converge to a single point. As shown in FIG. 4, if the ultrasound scanner is driven by a motor, the scanning can be performed while the outer surface of the bladder scanner is fixed to the patient's abdominal surface. However, as shown in FIG. 5, if the operator manually tilts the bladder scanner, the outer surface of the bladder scanner moves along the surface of the patient's abdomen. In particular, since the surface of the probe covering, which is the exterior of the bladder scanner, and the ultrasound transducer are physically separated, the movement of position of the ultrasound transducer occurs as the bottom of the probe covering moves. As a result, a problem arises in which the position of the ultrasound transducer is not fixed and moves spatially during ultrasound scanning due to manual rotation by the operator.

Therefore, when the bladder scanner acquires a plurality of two-dimensional ultrasound images through ultrasound scanning according to manual rotation by the operator and uses them to calculate the volume of urine, there is a problem in which errors may occur due to the unwanted positional movement of the ultrasound transducer.

## [DETAILED DESCRIPTION OF THE INVENTION]

## [TECHNICAL CHALLENGES]

The object of the present invention to solve the above-mentioned problems is to provide a method which reduces the measurement error of the volume of urine in a bladder scanner that uses mechanical rotation by a motor in one direction and manual rotation by the operator in the other direction.

Therefore, the bladder scanner according to the present invention is configured to correct the coordinates of the ultrasound echo signal by taking into account the unwanted physical position movement of the ultrasound transducer that occurs when calculating the volume of urine in the bladder, and measure the volume of urine through this coordinate correction in order to reduce the measurement errors in the volume of urine.

## [TECHNICAL SOLUTION]

According to one aspect of the present invention to achieve the above-mentioned technical solution, there is provided an ultrasound scanner which includes; an ultrasound transducer that transmits ultrasound signals to a measurement object and receives ultrasound echo signals reflected from the measurement object; a single motor connected to a central axis of the ultrasound transducer; an ultrasound probe with the ultrasound transducer and motor mounted therein; a tilt sensor mounted on the central axis of the ultrasound probe to detect and provide a tilt angle of the ultrasound probe with respect to a second direction; and a control unit acquiring sector-shaped 2D ultrasound images at a plurality of tilt angles in response to a change in tilt angle by manual manipulation, which the 2D ultrasound images are obtained by rotating the ultrasound transducer using the motor in a first direction perpendicular to the second direction, wherein the control unit receives the ultrasound echo signal from the ultrasound transducer, corrects the coordinates of the ultrasound echo signal by considering the tilt angle of the ultrasound probe by the manual manipulation, and acquires a sector-shaped 2D ultrasound image using the ultrasound echo signals with the corrected coordinates.

In the ultrasound scanner having the above-described features according to the present invention, it is preferable that the control unit includes: a coordinate correction module which receives the ultrasound echo signals from the ultrasound transducer, and corrects the coordinates of the ultrasound echo signals by considering the tilt angle of the ultrasound probe by manual manipulation; and a 2D ultrasound image acquisition module which rotates the ultrasound transducer along the first direction by driving a motor, acquires ultrasound echo signals according to the rotation movement, corrects the coordinates of the ultrasound echo signals using the coordinate correction module, and acquires a sector-shaped two-dimensional ultrasound image using the ultrasound echo signals with the corrected coordinates.

In the ultrasound scanner having the above-described feature according to the present invention, it is preferable that the control unit further includes a 3D information extraction module which acquires a plurality of 2D ultrasound images at a plurality of tilt angles by repeatedly driving the 2D ultrasound image acquisition module in response to changes in the tilt angle due to manual manipulation, and extracts a preset 3D information using the plurality of 2D ultrasound images.

In the ultrasound scanner having the above-described feature according to the present invention, it is preferable that the coordinate correction module receives an ultrasound echo signal from the ultrasound transducer, obtains the initial coordinates (x, y) for the ultrasound echo signal based on the origin preset as a reference position of the ultrasound transducer, calculates error values ($\Delta x$, $\Delta y$) indicating the degree to which the ultrasound transducer deviates from the origin using the tilt angle (θ) of the ultrasound probe provided from the tilt sensor, corrects the initial coordinates for the ultrasound echo signal using the error values, and provides corrected coordinates (x', y') for the ultrasound echo signal.

[EFFECTS OF THE INVENTION]

The conventional ultrasound scanners are configured to perform mechanical rotation using a motor in one direction and manual rotation by the operator in the other direction, Accordingly, the ultrasonic transducer deviates from the initial reference point, and as a result, the coordinates of the ultrasonic echo signal cannot be accurately calculated.

Therefore, the ultrasonic scanner according to the present invention calculates an error value due to the positional deviation of the ultrasonic transducer and corrects the coordinates of the ultrasonic echo signal using the error value. As a result, the ultrasound scanner according to the present invention can obtain accurate position coordinates for the ultrasound echo signal, thereby minimizing distortion of the ultrasound image.

Additionally, the ultrasound scanner according to the present invention can greatly reduce measurement errors in the volume of urine compared to conventional methods by minimizing distortion of ultrasound images.

[BRIEF DESCRIPTION OF THE DRAWINGS]

FIG. 1 is a perspective view showing a conventional bladder scanner configured to obtain a three-dimensional image using two motors.

FIG. 2 is a diagram shown to explain the rotation of the ultrasound transducer for a sector scan in the bladder scanner of FIG. 1.

FIG. 3 is a diagram showing a three-dimensional ultrasound scan trajectory using a sector scan in the bladder scanner of FIG. 1.

FIG. 4 is a schematic diagram showing the direction in which the motor rotates the bladder scanner to obtain a three-dimensional image in the bladder scanner of FIG. 1.

FIG. 5 is a schematic diagram showing the direction in which an operator manually rotates the bladder scanner left and right in a conventional bladder scanner that is configured to obtain a 3D image using a single motor.

FIG. 6 is a diagram showing an ideal three-dimensional ultrasound scan trajectory in the conventional bladder scanner according to FIG. 5.

FIG. 7 shows a moving trajectory of the ultrasound transducer in the bladder scanner according to FIG. 5 when the operator tilts the bladder scanner by hand.

FIG. 8 shows an actual scan trajectory of the bladder scanner according to FIG. 5 when the operator moves the bladder scanner by hand.

FIG. 9 is a graph illustrating an ultrasonic echo signal as an example.

FIG. 10 is a graph shown to explain coordinates in a mechanical scanning method that rotates an ultrasound transducer using a motor in a bladder scanner.

FIG. 11 is a graph shown to explain coordinates in a scanning method in which an operator manually rotates the bladder scanner in the bladder scanner according to FIG. 5.

FIG. 12 is a block diagram schematically showing the structure of an ultrasound scanner according to a preferred embodiment of the present invention.

[BEST MODE FOR CARRYING OUT THE INVENTION]

Hereinafter, an ultrasound scanner and a position correction method in the ultrasound scanner according to a preferred embodiment of the present invention will be described in more detail with reference to the attached drawings.

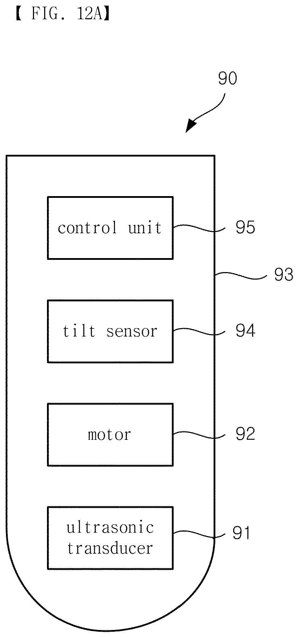

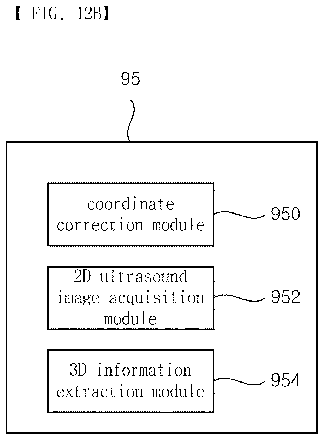

FIG. 12 is a block diagram showing an ultrasound scanner according to a preferred embodiment of the present invention. Referring to FIG. 12, the ultrasound scanner 90 according to the present invention includes an ultrasound probe 93 and a control unit 95. The ultrasound probe 93 is equipped with an ultrasound transducer 91 composed of a single element, a motor 92, and a tilt sensor 94. The control unit may be composed of a microprocessor or the like and may be mounted inside the ultrasound probe or may be mounted on a separate device and physically connected to the ultrasound probe.

The ultrasound transducer 91, which is composed of a single element, transmits an ultrasound signal to a measurement object and receives an ultrasound echo signal reflected from the measurement object. The motor 92 is connected to the central axis of the ultrasound transducer and rotates the ultrasound transducer in a first direction under the control of the control unit. The ultrasound probe 93 is equipped with the ultrasound transducer, motor, and tilt sensor inside. The tilt sensor 94 is mounted on the central axis of the ultrasound probe to detect a tilt angle of the ultrasound probe with respect to the second direction. The second direction is perpendicular to the first direction.

The control unit 95 includes a coordinate correction module 950, a 2D ultrasound image acquisition module 952, and a 3D information extraction module 954. The control unit rotates the ultrasound transducer along a first direction using the motor to obtain a sector-shaped two-dimensional ultrasound image which is a sector image. The control unit acquires a plurality of two-dimensional ultrasound images at a plurality of tilt angles in response to a change in tilt angle due to the manual movement of the operator. The control unit extracts preset 3D information using a plurality of 2D ultrasound images. Here, the first direction and the second direction are perpendicular to each other. While the operator manually rotates the ultrasound probe along the second direction, the control unit drives the motor to repeatedly rotate and scan the ultrasound transducer in the ultrasound probe along the first direction, in order to acquire a plurality of two-dimensional ultrasound images. In particular, when the ultrasound echo signals are received from the ultrasound transducer, the control unit 95 corrects the coordinates of the ultrasound echo signals by considering the tilt angle of the ultrasound probe by manual operation of the operator, and generates a sector-shaped two-dimensional ultrasound image using the ultrasound echo signals with the corrected coordinates.

The coordinate correction module 950 is a module for correcting physical position errors that occur because the lower portion of the covering of the ultrasound probe in contact with the measurement point and the ultrasound transducer, which is the measurement sensor, are physically spaced apart from each other. The coordinate correction module 950 receives an ultrasound echo signal, obtains the initial coordinates (x, y) of the ultrasound echo signal based on the origin preset as the reference position of the ultrasound transducer. Next, using the tilt angle (θ) of the ultrasound probe provided from the tilt sensor, error values (Δx, Δy) indicating the degree to which the ultrasound transducer deviates from the origin are calculated. Next, the initial coordinates for the ultrasound echo signal are corrected using the error values, and the corrected coordinates (x', y') for the ultrasound echo signal are provided. The coordinates (x', y') corrected by the coordinate correction module according to the above-described process are obtained by the equations below.

【 EQUATION 1】

$$x' = x - \Delta x$$

【 EQUATION 2】

$$y' = y + \Delta y$$

【 EQUATION 3】

$$\Delta y = \frac{\Delta d}{\cos\theta} - \Delta x \tan\theta$$

【 EQUATION 4】

$$\Delta x = R(\theta - \sin\theta)$$

【 EQUATION 5】

$$\Delta d = R(\frac{\theta}{\sin\theta} + \cos\theta - 1 + \theta\frac{\cos^2\theta}{\sin\theta})$$

Here, (x,y) is the initial coordinate for the ultrasound echo signal based on the origin, (x',y') is the corrected coordinate for the ultrasound echo signal considering the tilt angle of the ultrasound probe, Δd is the amount of movement of the ultrasound echo signal in the axial direction, and R represents the radius of the covering at the bottom of the ultrasound probe.

The 2D ultrasound image acquisition module 952 drives a motor to rotate the ultrasound transducer along a first direction perpendicular to the second direction, acquires ultrasound echo signals according to the rotational movement, corrects the coordinates of the ultrasound echo signals using the coordinate correction module, and acquires a sector-shaped two-dimensional ultrasound image using the ultrasound echo signals with the corrected coordinates.

The 3D information extraction module 954 repeatedly drives the 2D ultrasound image acquisition module 952 in response to a change in tilt angle due to manual manipulation by the operator, thereby acquiring 2D ultrasound images at a plurality of tilt angles. Next, the 3D information extraction module 954 extracts preset 3D information using the obtained plurality of 2D ultrasound images. The three-dimensional information is characterized by the volume of urine obtained from the volume of the bladder.

Hereinafter, the operation of the bladder scanner, which is an ultrasound scanner according to the present invention, having the above-described configuration will be described in more detail. The bladder scanner according to the present invention performs ultrasound scanning using the mechanical rotation by driving the motor in the first direction using a single motor, while rotating in the second direction by manual manipulation of the operator in the second direction,

FIG. 5 is a schematic diagram showing the process of scanning the bladder scanner with a single motor according to the present invention while the operator manually rotates it in the left and right directions. Referring to FIG. 5, the operator holds the bladder scanner with his hand and tilts the bladder scanner to the left and right to perform the ultrasound scan. The motor drive unit built into the bladder scanner performs a sector scan like conventional technology, and the sector scan is performed in the up and down directions.

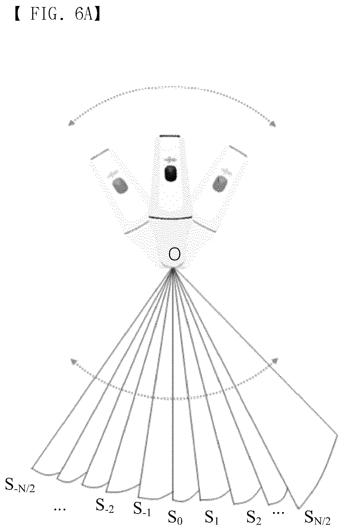

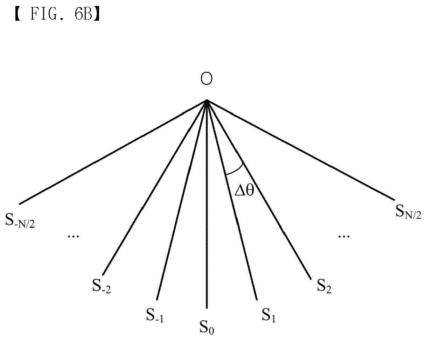

FIGS. 6A and 6B illustrate an ideal three-dimensional ultrasound scan trajectory in a bladder scanner with a single motor according to the present invention. If the tilt angle according to manual rotation by the operator is θ, scan planes as shown in FIG. 6A are obtained. In FIG. 6A, each sector-shaped scan plane is obtained by ultrasound scanning according to rotation of the ultrasound transducer by the motor. And, the set of the scan planes can be obtained by manual rotation of the operator. If the plane of the sector scan of FIG. 6B is marked with a line, a set of scan planes like that of FIG. 6A can be obtained. One line in FIG. 6B corresponds to one scan plane in FIG. 6A. It is desirable that the separation angle $\Delta\theta$ of adjacent scan planes is constant. However, because it is difficult to manually rotate the ultrasound scanner at a constant angular speed, the separation angle $\Delta\theta$ may vary for each scanning plane. However, if the value of separation angle $\Delta\theta$ is known, the 3D volume image of the ultrasound image can be obtained by using the separation angle $\Delta\theta$. By attaching an angle sensor to the upper part of the bladder scanner, the scan angle (θ) when acquiring each plane of the sector scan can be obtained, and thus the separation angle ($\Delta\theta$) of adjacent scan planes can also be obtained.

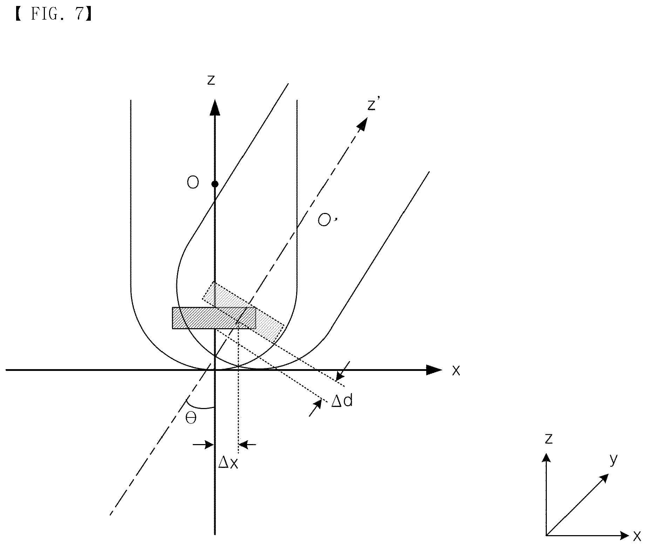

In FIGS. 6A and 6B, each scan plane is indicated by $S_i$, and the number of scan planes is N+1. As the number of scanning planes increases, the resolution of the 3D volume image improves, but the time required to obtain the 3D volume image increases. Considering this, it is generally recommended that the number of the scan planes be 10 to 20. In FIG. 6B, all scan lines are assumed to come from the point 0, but this is not the case in reality. Referring to FIG. 7 while performing a sector scan using a motor mounted inside the probe of the bladder scanner, if the operator uses his hand to tilt the probe in a direction perpendicular to the sector scan, another sector scan is performed along the tilted direction.

FIG. 7 shows a moving trajectory of the ultrasound transducer when manually tilting the bladder scanner according to the present invention. Assume that a sector scan is performed using a motor along the z-axis direction, and the bladder scanner is rotated by hand along the x-axis direction. When the ultrasound probe is tilted in the x-axis direction, unlike sector scan by a motor, the position of the ultrasound transducer is not fixed at point 0 but changes depending on the tilt angle (θ). This is because, as the bladder scanner is tilted, the hemispherical lower part of the bladder scanner moves in the x-axis direction while keeping contact with the patient's abdominal surface. That is, the contact point between the lower end of the bladder scanner (i.e., the lower end of the covering of the ultrasound probe) and the patient's abdominal surface is not fixed but moves in the x-axis direction. Good contact must be maintained between the lower end of the ultrasound probe and the patient's abdominal surface to prevent an air layer from forming. Otherwise, ultrasound beams are excessively reflected from the air layer, making it impossible to obtain high-quality ultrasound images.

When the operator manually tilts the bladder scanner, the position of the transducer not only moves in the x-axis (right and left) direction, but also changes in the y-axis (height) direction, as shown in FIG. 7. This is because there must be a separation distance having certain distance between the ultrasound transducer and the probe covering for sector scan by the motor. This separation distance is usually about a few millimeters. Therefore, the position (x, y) of the transducer is not fixed and changes depending on the angle at which the operator manually tilts the probe. In this situation, if ultrasound echo signals are collected and an image is generated assuming an ideal sector scan as shown in FIG. 6, the image will be distorted. If the degree of image distortion is not severe, it may be difficult to recognize the distortion when viewing the ultrasound image with the naked eye. However, when estimating the volume of urine from the collected ultrasound images, the estimation error can become very large. For example, if the distortion in one direction of the image is at the level of 2%, the error in volume calculation can increase to the cube of the amount of distortion, or 8%. Therefore, the actual trajectory of the ultrasound scan must be taken into account when calculating the volume of urine.

In FIG. 7, when the ultrasound probe is tilted by θ, the movement amount $\Delta x$ in the x-axis direction of the ultrasound probe and the movement amount $\Delta d$ in the axial direction of the bladder scanner, that is, in the z' direction, are expressed by the equations below.

【 EQUATION 6】

$$\Delta x = R(\theta - \sin\theta)$$

【 EQUATION 7】

$$\Delta d = R\left(\frac{\theta}{\sin\theta} + \cos\theta - 1 + \theta\frac{\cos^2\theta}{\sin\theta}\right)$$

In the above equation, R represents the radius of the covering at the bottom of the ultrasound probe, and θ is the tilt angle of the ultrasound probe due to manual movement.

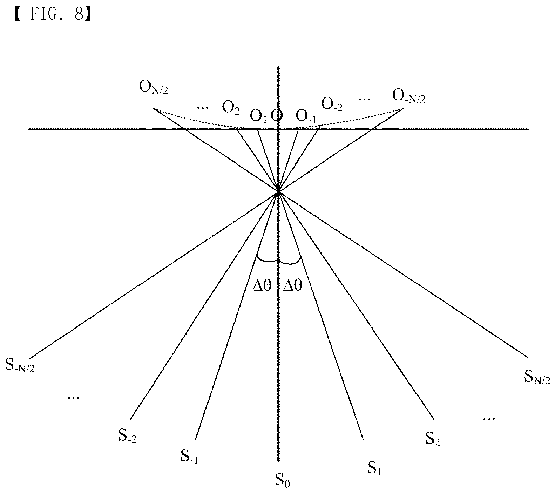

FIG. 8 shows an actual scan trajectory in the bladder scanner according to the present invention when the bladder scanner is moved by the operator's hand. The scan plane considering $\Delta x$ and $\Delta d$ is as shown in FIG. 8. Now, the ultrasound scan plane does not converge to one-point 0, but changes by the Equation 6 and Equation 7 according to the tilt angle θ. Since the convergence point of each sector scan plane is different, this must be taken into consideration when obtaining a 3D volume image. Through these considerations, the distortion of the ultrasound image can be eliminated and the bladder volume can be measured without error.

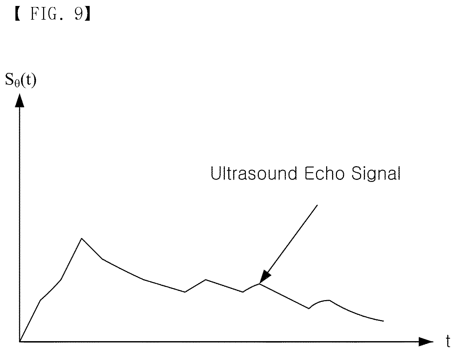

FIG. 9 shows the waveform of the ultrasound echo signal received by the ultrasound transducer in the bladder scanner according to the present invention. The ultrasound echo signal received at a scan angle of $\theta$ is denoted as $S_\theta(t)$.

The depth r at which the ultrasound echo signal is generated satisfies Equation 8.

【 EQUATION 8】

$$r = ct/2$$

Here, c is the sonic speed within the human body and is approximately 1500 m/s within the human soft tissue. Since time t and depth r have a linear relationship, the ultrasound echo signal is a function of time and depth at the same time, expressed by Equation 9.

【 EQUATION 9】

$$S_\theta(t) = S_\theta(2r/c) = S_\theta{}'(r)$$

When performing mechanical scanning as shown in FIG. 3, all scan lines converge to the point 0.

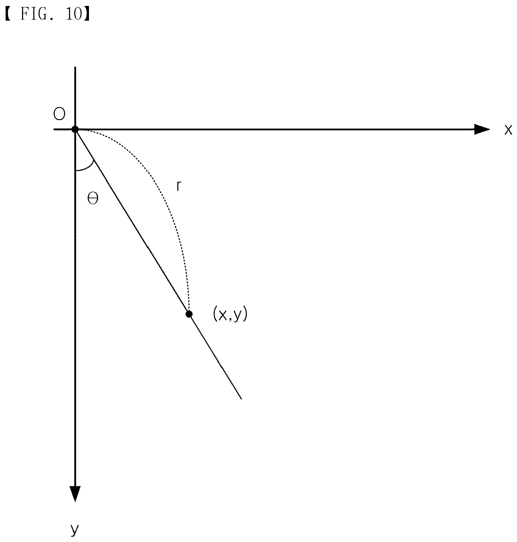

FIG. 10 is a coordinate in a mechanical scanning method, showing scan lines collected at an angle $\theta$. Since the scan line corresponds to polar coordinates with respect to the xy-plane, the ultrasound echo signal at the position of depth r corresponds to the ultrasound image I(x,y) in the rectangular coordinate system using Equation 10.

【 EQUATION 10】

$$I(x, y) = \left| S_\theta{}'(r) \right|_{x=rsin\theta, y=rcos\theta}$$

By converting ultrasound echo signals at all angles using Equation 10, an ultrasound image in a rectangular coordinate system can be obtained.

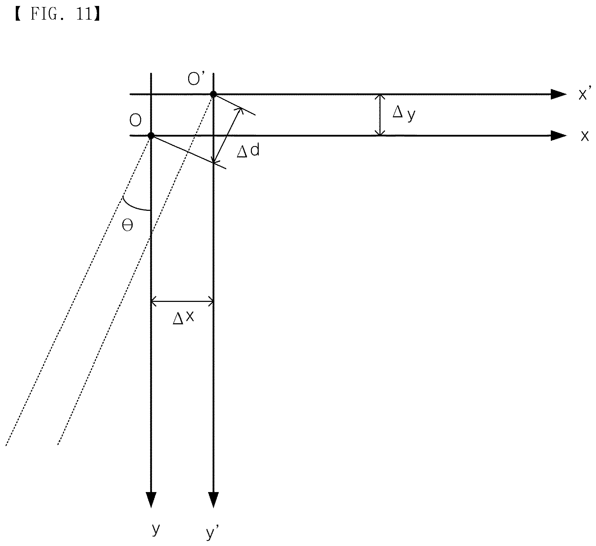

In the case of ultrasound scan based on manual rotation by an operator, as described above, the scan lines do not converge at one point and produce displacements of $\Delta$d and $\Delta$x. FIG. 11 shows the xy coordinate system of ultrasound scanning according to mechanical rotation and the x'y' coordinate system of ultrasound scanning according to manual rotation in the ultrasound scanner according to the present invention. In ultrasound scanning based on mechanical rotation, scan lines converge around point 0, so the xy-plane is fixed at all angles. However, in ultrasound scanning based on manual rotation by the operator, the x'y' plane moves in parallel depending on the angle. (x,y) and (x', y') can be expressed as Equations 11 to 13.

【 EQUATION 11】

$$x' = x - \Delta x$$

【 EQUATION 12】

$$y' = y + \Delta y$$

【 EQUATION 13】

$$\Delta y = \frac{\Delta d}{cos\theta} - \Delta x \, tan\theta$$

The ultrasound echo signal obtained at the angle $\theta$ using an ultrasound scanning method based on manual rotation is expressed as $\tilde{S}_\theta(r)$. The ultrasound image signal obtained by coordinate transformation of this ultrasound echo signal is expressed by Equation 14.

【 EQUATION 14】

$$\tilde{I}(x', y') = \left| \tilde{S}_\theta{}'(r) \right|_{x'=rsin\theta, y'=rcos\theta}$$

Since the (x',y') coordinate system changes depending on the angle, the final ultrasound image signal obtained by converting it to the fixed coordinate system (x,y) is expressed in Equation 15.

【 EQUATION 15】

$$I(x, y) = I(x' + \Delta x, y' - \Delta y)$$

Although the present invention has been described above with a focus on preferred embodiments, this is only an example and does not limit the present invention, and those skilled in the art will understand that it does not deviate from the essential characteristics of the present invention. It will be apparent that various modifications and applications not exemplified above are possible within the scope. In addition, the differences in these variations and application should be construed as being included in the scope of the present invention as defined in the appended claims.

**Claims**

1. An ultrasound scanner includes;

   an ultrasound transducer that transmits ultrasound signals to a measurement object and receives ultrasound echo signals reflected from the measurement object;
   a single motor connected to a central axis of the ultrasound transducer;
   an ultrasound probe with the ultrasound transducer and motor mounted therein;
   a tilt sensor mounted on a central axis of the ultrasound probe to detect a tilt angle of the ultrasound probe with respect to a second direction; and
   a control unit acquiring sector-shaped 2D ultrasound images at a plurality of tilt angles in response to a change in tilt angle by manual manipulation, which the 2D ultrasound images are obtained by rotating the ultrasound transducer using the motor in a first direction perpendicular to the second direction,
   wherein the control unit receives the ultrasound echo signals from the ultrasound transducer, corrects the coordinates of the ultrasound echo signals by considering the tilt angle of the ultrasound probe by the manual manipulation, and acquires a sector-shaped 2D ultrasound image using the ultrasound echo signals with the corrected coordinates.

2. The ultrasound scanner according to claim 1, wherein the control unit includes:

   a coordinate correction module that receives the ultrasound echo signals from the ultrasound transducer, and corrects the coordinates of the ultrasound echo signals by considering the tilt angle of the ultrasound probe by manual manipulation; and
   a 2D ultrasound image acquisition module that rotates the ultrasound transducer along the first direction by driving the motor, acquires ultrasound echo signals according to the rotation movement, corrects the coordinates of the ultrasound echo signals using the coordinate correction module, and acquires a sector-shaped two-dimensional ultrasound image using the ultrasound echo signals with the corrected coordinates.

3. The ultrasound scanner according to claim 2, wherein the coordinate correction module

   receives ultrasound echo signals from the ultrasound transducer,
   obtains the initial coordinates (x, y) for the ultrasound echo signals based on the origin preset as a reference position of the ultrasound transducer,
   calculates error values ($\Delta$x, $\Delta$y) indicating the degree to which the ultrasound transducer deviates from the origin using the tilt angle ($\theta$) of the ultrasound probe provided from the tilt sensor,
   corrects the initial coordinates for the ultrasound echo signals using the error values, and
   provides corrected coordinates (x', y') for the ultrasound echo signals.

4. The ultrasound scanner according to claim 3, wherein the corrected coordinates (x', y') of the coordinate correction module are obtained by the equations below,

$$x' = x - \Delta x \quad,$$

$$y' = y + \Delta y \quad,$$

$$\Delta y = \frac{\Delta d}{\cos\theta} - \Delta x \tan\theta \ ,$$

$$\Delta x = R(\theta - \sin\theta),$$

and

$$\Delta d = R(\frac{\theta}{\sin\theta} + \cos\theta - 1 + \theta\frac{\cos^2\theta}{\sin\theta}) \ ,$$

here, (x,y) is the initial coordinate for the ultrasound echo signal based on the origin, (x',y') is the corrected coordinate for the ultrasound echo signal considering the tilt of the ultrasound probe, and $\Delta$d is the amount of movement in the axial direction of the ultrasound probe, and R is a radius of the lower covering of the ultrasound probe.

5. The ultrasound scanner according to claim 2, wherein the control unit further includes a 3D information extraction module that acquires a plurality of 2D ultrasound images at a plurality of tilt angles by repeatedly executing the 2D ultrasound image acquisition module in response to changes in the tilt angle due to manual manipulation, and extracts a preset 3D information using the plurality of 2D ultrasound images.

6. The ultrasound scanner according to claim 5, wherein the 3D information extracted by the control unit is volume of urine calculated from volume of a bladder.

【FIG. 1】

【FIG. 2】

【 FIG. 3A】

【 FIG. 3B 】

【 FIG. 4】

【FIG. 5】

【FIG. 6A】

$S_{-N/2}$ ... $S_{-2}$ $S_{-1}$ $S_0$ $S_1$ $S_2$ ... $S_{N/2}$

【FIG. 6B】

O

$\Delta\theta$

$S_{-N/2}$

...

$S_{-2}$

$S_{-1}$

$S_0$

$S_1$

$S_2$

...

$S_{N/2}$

【FIG. 7】

【FIG. 8】

【 FIG. 9】

【FIG. 10】

【FIG. 11】

【FIG. 12A】

90

control unit — 95

93

tilt sensor — 94

motor — 92

ultrasonic
transducer — 91

【 FIG. 12B】

95

```
┌─────────────────────────────────────┐
│                                      │
│   ┌──────────────────────┐           │
│   │      coordinate      │           │
│   │  correction module   │  ～ 950   │
│   └──────────────────────┘           │
│                                      │
│   ┌──────────────────────┐           │
│   │    2D ultrasound      │          │
│   │  image acquisition    │  ～ 952   │
│   │       module          │          │
│   └──────────────────────┘           │
│                                      │
│   ┌──────────────────────┐           │
│   │   3D information      │          │
│   │  extraction module    │  ～ 954   │
│   └──────────────────────┘           │
│                                      │
└─────────────────────────────────────┘
```

## INTERNATIONAL SEARCH REPORT

| International application No. |
|---|
| **PCT/KR2021/019851** |

**A. CLASSIFICATION OF SUBJECT MATTER**

**A61B 8/14**(2006.01)i; **A61B 8/08**(2006.01)i

According to International Patent Classification (IPC) or to both national classification and IPC

**B. FIELDS SEARCHED**

Minimum documentation searched (classification system followed by classification symbols)

A61B 8/14(2006.01); A61B 8/00(2006.01); A61B 8/08(2006.01); A61F 5/44(2006.01); G01N 29/24(2006.01); G01S 15/89(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 초음파(ultrasound), 트랜스듀서(transducer), 모터(motor), 좌표(coordinate), 보정(calibration), 2차원(two-dimensions), 3차원(three-dimensions)

**C. DOCUMENTS CONSIDERED TO BE RELEVANT**

| Category* | Citation of document, with indication, where appropriate, of the relevant passages | Relevant to claim No. |
|---|---|---|
| X | KR 10-2333542 B1 (SAMSUNG MEDISON CO., LTD.) 01 December 2021 (2021-12-01)<br>See paragraphs [0018] and [0080]-[0094]; claims 1 and 2; and figures 8a and 8b. | 1,2,5 |
| Y | | 3,4,6 |
| Y | JP 2004-516865 A (SONOCINE, INC.) 10 June 2004 (2004-06-10)<br>See paragraphs [0036] and [0037]. | 3,4 |
| Y | KR 10-2333542 A (MCUBETECHNOLOGY CO., LTD.) 30 October 2007 (2007-10-30)<br>See paragraph [0078]. | 6 |
| A | US 2004-0267123 A1 (MCMORROW et al.) 30 December 2004 (2004-12-30)<br>See entire document. | 1-6 |
| A | JP 2003-190168 A (KOYAMA, Jun et al.) 08 July 2003 (2003-07-08)<br>See entire document. | 1-6 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **30 September 2022** | **04 October 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

<div align="center">

**INTERNATIONAL SEARCH REPORT**
**Information on patent family members**

</div>

International application No.

**PCT/KR2021/019851**

| Patent document cited in search report | | | Publication date (day/month/year) | Patent family member(s) | | | Publication date (day/month/year) |
|---|---|---|---|---|---|---|---|
| KR | 10-2333542 | B1 | 01 December 2021 | EP | 3020336 | A1 | 18 May 2016 |
| | | | | EP | 3020336 | B1 | 29 November 2017 |
| | | | | US | 2016-0135785 | A1 | 19 May 2016 |
| | | | | US | 9962140 | B2 | 08 May 2018 |
| JP | 2004-516865 | A | 10 June 2004 | AT | 291372 | T | 15 April 2005 |
| | | | | AU | 2002-13325 | A1 | 22 April 2002 |
| | | | | AU | 2002-213325 | B2 | 27 July 2006 |
| | | | | BR | 0114815 | A | 03 February 2004 |
| | | | | BR | 0114815 | B1 | 18 October 2011 |
| | | | | CA | 2424077 | A1 | 18 April 2002 |
| | | | | CA | 2424077 | C | 12 April 2016 |
| | | | | CA | 2773055 | A1 | 18 April 2002 |
| | | | | CA | 2773055 | C | 08 October 2013 |
| | | | | CN | 1215817 | C | 24 August 2005 |
| | | | | CN | 1469723 | A | 21 January 2004 |
| | | | | DE | 60109629 | T2 | 09 February 2006 |
| | | | | EP | 1324700 | A2 | 09 July 2003 |
| | | | | EP | 1324700 | B1 | 23 March 2005 |
| | | | | ES | 2238052 | T3 | 16 August 2005 |
| | | | | IL | 155359 | A | 03 December 2007 |
| | | | | JP | 3735605 | B2 | 18 January 2006 |
| | | | | US | 2003-0125623 | A1 | 03 July 2003 |
| | | | | US | 2003-0125625 | A1 | 03 July 2003 |
| | | | | US | 2003-0125626 | A1 | 03 July 2003 |
| | | | | US | 2004-0015080 | A1 | 22 January 2004 |
| | | | | US | 2006-0036173 | A1 | 16 February 2006 |
| | | | | US | 2007-0073149 | A1 | 29 March 2007 |
| | | | | US | 2015-0099974 | A1 | 09 April 2015 |
| | | | | US | 6524246 | B1 | 25 February 2003 |
| | | | | US | 6733448 | B2 | 11 May 2004 |
| | | | | US | 6808495 | B2 | 26 October 2004 |
| | | | | US | 6932768 | B2 | 23 August 2005 |
| | | | | US | 7445599 | B2 | 04 November 2008 |
| | | | | US | 7556603 | B2 | 07 July 2009 |
| | | | | US | 8911370 | B2 | 16 December 2014 |
| | | | | US | 9486181 | B2 | 08 November 2016 |
| | | | | WO | 02-030287 | A3 | 13 March 2003 |
| | | | | WO | 02-30287 | A2 | 18 April 2002 |
| KR | 10-2007-0105097 | A | 30 October 2007 | CA | 2624651 | A1 | 01 November 2007 |
| | | | | CA | 2624651 | C | 24 February 2015 |
| | | | | CN | 101355905 | A | 28 January 2009 |
| | | | | CN | 101355905 | B | 02 February 2011 |
| | | | | EP | 2010060 | A1 | 07 January 2009 |
| | | | | EP | 2010060 | B1 | 09 February 2011 |
| | | | | EP | 2010060 | B9 | 31 August 2011 |
| | | | | JP | 2009-512532 | A | 26 March 2009 |
| | | | | JP | 5426169 | B2 | 26 February 2014 |
| | | | | US | 2009-0030326 | A1 | 29 January 2009 |
| | | | | US | 2011-0137172 | A1 | 09 June 2011 |
| | | | | US | 2014-0024937 | A1 | 23 January 2014 |

Form PCT/ISA/210 (patent family annex) (July 2019)

**INTERNATIONAL SEARCH REPORT**
Information on patent family members

International application No.

**PCT/KR2021/019851**

| Patent document cited in search report | | | Publication date (day/month/year) | Patent family member(s) | | | Publication date (day/month/year) |
|---|---|---|---|---|---|---|---|
| | | | | US | 2016-0192904 | A1 | 07 July 2016 |
| | | | | US | 2016-0367218 | A1 | 22 December 2016 |
| | | | | WO | 2007-123352 | A1 | 01 November 2007 |
| US | 2004-0267123 | A1 | 30 December 2004 | AT | 493933 | T | 15 January 2011 |
| | | | | AT | 505135 | T | 15 April 2011 |
| | | | | AT | 510499 | T | 15 June 2011 |
| | | | | AT | 512628 | T | 15 July 2011 |
| | | | | AU | 2003-237819 | A1 | 22 December 2003 |
| | | | | AU | 2003-254372 | A1 | 11 March 2004 |
| | | | | AU | 2003-261357 | A1 | 23 February 2004 |
| | | | | AU | 2003-296928 | A1 | 07 June 2004 |
| | | | | CA | 2526297 | A1 | 18 December 2003 |
| | | | | CA | 2534287 | A1 | 12 February 2004 |
| | | | | CA | 2541798 | A1 | 21 May 2004 |
| | | | | CA | 2587137 | A1 | 19 May 2005 |
| | | | | CA | 2598335 | A1 | 01 September 2005 |
| | | | | CA | 2616541 | A1 | 08 February 2007 |
| | | | | CA | 2617622 | A1 | 09 March 2006 |
| | | | | CA | 2617622 | C | 31 May 2016 |
| | | | | CA | 2626226 | A1 | 04 May 2006 |
| | | | | CA | 2631937 | A1 | 15 June 2006 |
| | | | | CA | 2665165 | A1 | 21 August 2008 |
| | | | | CA | 2665165 | C | 16 December 2014 |
| | | | | CA | 2671708 | A1 | 10 July 2008 |
| | | | | CA | 2688758 | A1 | 27 November 2008 |
| | | | | CA | 2688758 | C | 05 July 2016 |
| | | | | CA | 2688778 | A1 | 27 November 2008 |
| | | | | CA | 2688778 | C | 14 April 2020 |
| | | | | CA | 2732997 | A1 | 11 February 2010 |
| | | | | CA | 2732997 | C | 14 March 2017 |
| | | | | CA | 2957778 | A1 | 11 February 2010 |
| | | | | CN | 101553579 | A | 07 October 2009 |
| | | | | CN | 101553579 | B | 20 January 2016 |
| | | | | EP | 1521548 | A1 | 13 April 2005 |
| | | | | EP | 1538986 | A2 | 15 June 2005 |
| | | | | EP | 1538986 | B1 | 15 June 2011 |
| | | | | EP | 1551305 | A1 | 13 July 2005 |
| | | | | EP | 1551305 | B1 | 23 December 2015 |
| | | | | EP | 1596718 | A2 | 23 November 2005 |
| | | | | EP | 1689298 | A1 | 16 August 2006 |
| | | | | EP | 1781176 | A2 | 09 May 2007 |
| | | | | EP | 1781176 | B1 | 13 April 2011 |
| | | | | EP | 1784129 | A2 | 16 May 2007 |
| | | | | EP | 1784129 | B1 | 25 May 2011 |
| | | | | EP | 1809178 | A2 | 25 July 2007 |
| | | | | EP | 1819279 | A2 | 22 August 2007 |
| | | | | EP | 1819279 | B1 | 05 January 2011 |
| | | | | EP | 1909648 | A2 | 16 April 2008 |
| | | | | EP | 2097009 | A2 | 09 September 2009 |
| | | | | EP | 2155067 | A2 | 24 February 2010 |

Form PCT/ISA/210 (patent family annex) (July 2019)

INTERNATIONAL SEARCH REPORT
Information on patent family members

International application No.

**PCT/KR2021/019851**

| Patent document cited in search report | Publication date (day/month/year) | Patent family member(s) | | Publication date (day/month/year) |
|---|---|---|---|---|
| | | EP | 2155067 B1 | 06 January 2016 |
| | | EP | 2155068 A1 | 24 February 2010 |
| | | EP | 2155068 B1 | 21 December 2016 |
| | | EP | 2164988 A2 | 24 March 2010 |
| | | EP | 2164988 B1 | 17 February 2016 |
| | | EP | 2186483 A1 | 19 May 2010 |
| | | EP | 2186483 B1 | 24 June 2020 |
| | | EP | 2323559 A1 | 25 May 2011 |
| | | GB | 2391625 A | 11 February 2004 |
| | | JP | 2005-528950 A | 29 September 2005 |
| | | JP | 2005-534462 A | 17 November 2005 |
| | | JP | 2005-535420 A | 24 November 2005 |
| | | JP | 2007-522884 A | 16 August 2007 |
| | | JP | 2007-524474 A | 30 August 2007 |
| | | JP | 2008-511408 A | 17 April 2008 |
| | | JP | 2008-517673 A | 29 May 2008 |
| | | JP | 2008-522661 A | 03 July 2008 |
| | | JP | 2009-279435 A | 03 December 2009 |
| | | JP | 2009-502354 A | 29 January 2009 |
| | | JP | 2010-207596 A | 24 September 2010 |
| | | JP | 2010-514524 A | 06 May 2010 |
| | | JP | 2010-527277 A | 12 August 2010 |
| | | JP | 2010-527278 A | 12 August 2010 |
| | | JP | 2010-540885 A | 24 December 2010 |
| | | JP | 2011-530352 A | 22 December 2011 |
| | | JP | 2012-101103 A | 31 May 2012 |
| | | JP | 2012-101104 A | 31 May 2012 |
| | | JP | 2015-042279 A | 05 March 2015 |
| | | JP | 4430532 B2 | 10 March 2010 |
| | | JP | 4549853 B2 | 22 September 2010 |
| | | JP | 5406272 B2 | 05 February 2014 |
| | | JP | 5420414 B2 | 19 February 2014 |
| | | JP | 5443883 B2 | 19 March 2014 |
| | | JP | 5538997 B2 | 02 July 2014 |
| | | JP | 5632278 B2 | 26 November 2014 |
| | | JP | 5646447 B2 | 24 December 2014 |
| | | JP | 5658151 B2 | 21 January 2015 |
| | | JP | 6162094 B2 | 12 July 2017 |
| | | KR | 10-0941208 B1 | 10 February 2010 |
| | | KR | 10-2004-0057077 A | 02 July 2004 |
| | | US | 10126302 B2 | 13 November 2018 |
| | | US | 2003-0229281 A1 | 11 December 2003 |
| | | US | 2004-0024302 A1 | 05 February 2004 |
| | | US | 2004-0024315 A1 | 05 February 2004 |
| | | US | 2004-0121578 A1 | 24 June 2004 |
| | | US | 2004-0127796 A1 | 01 July 2004 |
| | | US | 2004-0127797 A1 | 01 July 2004 |
| | | US | 2005-0215896 A1 | 29 September 2005 |
| | | US | 2005-0228278 A1 | 13 October 2005 |
| | | US | 2005-0251039 A1 | 10 November 2005 |

Form PCT/ISA/210 (patent family annex) (July 2019)

## INTERNATIONAL SEARCH REPORT
### Information on patent family members

| International application No. |
| --- |
| **PCT/KR2021/019851** |

| Patent document cited in search report | Publication date (day/month/year) | Patent family member(s) | | | Publication date (day/month/year) |
| --- | --- | --- | --- | --- | --- |
| | | US | 2006-0006765 | A1 | 12 January 2006 |
| | | US | 2006-0025689 | A1 | 02 February 2006 |
| | | US | 2006-0079775 | A1 | 13 April 2006 |
| | | US | 2006-0111633 | A1 | 25 May 2006 |
| | | US | 2006-0235301 | A1 | 19 October 2006 |
| | | US | 2007-0004983 | A1 | 04 January 2007 |
| | | US | 2007-0100236 | A1 | 03 May 2007 |
| | | US | 2007-0232908 | A1 | 04 October 2007 |
| | | US | 2007-0276247 | A1 | 29 November 2007 |
| | | US | 2007-0276254 | A1 | 29 November 2007 |
| | | US | 2008-0139934 | A1 | 12 June 2008 |
| | | US | 2008-0139938 | A1 | 12 June 2008 |
| | | US | 2008-0146932 | A1 | 19 June 2008 |
| | | US | 2008-0146939 | A1 | 19 June 2008 |
| | | US | 2008-0181479 | A1 | 31 July 2008 |
| | | US | 2008-0242985 | A1 | 02 October 2008 |
| | | US | 2008-0249414 | A1 | 09 October 2008 |
| | | US | 2008-0262356 | A1 | 23 October 2008 |
| | | US | 2008-0293164 | A1 | 27 November 2008 |
| | | US | 2009-0062644 | A1 | 05 March 2009 |
| | | US | 2009-0105585 | A1 | 23 April 2009 |
| | | US | 2009-0112089 | A1 | 30 April 2009 |
| | | US | 2009-0264757 | A1 | 22 October 2009 |
| | | US | 2010-0036242 | A1 | 11 February 2010 |
| | | US | 2010-0036252 | A1 | 11 February 2010 |
| | | US | 2010-0198075 | A1 | 05 August 2010 |
| | | US | 2010-0204581 | A1 | 12 August 2010 |
| | | US | 2011-0004101 | A1 | 06 January 2011 |
| | | US | 2012-0028828 | A1 | 02 February 2012 |
| | | US | 2012-0029155 | A1 | 02 February 2012 |
| | | US | 2015-0309016 | A1 | 29 October 2015 |
| | | US | 2016-0266132 | A1 | 15 September 2016 |
| | | US | 6676605 | B2 | 13 January 2004 |
| | | US | 6803308 | B2 | 12 October 2004 |
| | | US | 6884217 | B2 | 26 April 2005 |
| | | US | 7004904 | B2 | 28 February 2006 |
| | | US | 7041059 | B2 | 09 May 2006 |
| | | US | 7087022 | B2 | 08 August 2006 |
| | | US | 7450746 | B2 | 11 November 2008 |
| | | US | 7520857 | B2 | 21 April 2009 |
| | | US | 7611466 | B2 | 03 November 2009 |
| | | US | 7727150 | B2 | 01 June 2010 |
| | | US | 7744534 | B2 | 29 June 2010 |
| | | US | 7749165 | B2 | 06 July 2010 |
| | | US | 7819806 | B2 | 26 October 2010 |
| | | US | 8016760 | B2 | 13 September 2011 |
| | | US | 8133181 | B2 | 13 March 2012 |
| | | US | 8158444 | B2 | 17 April 2012 |
| | | US | 8167803 | B2 | 01 May 2012 |
| | | US | 8221321 | B2 | 17 July 2012 |

Form PCT/ISA/210 (patent family annex) (July 2019)

**INTERNATIONAL SEARCH REPORT**
Information on patent family members

| International application No. |
| --- |
| **PCT/KR2021/019851** |

| Patent document cited in search report | Publication date (day/month/year) | Patent family member(s) | | | Publication date (day/month/year) |
| --- | --- | --- | --- | --- | --- |
| | | US | 8221322 | B2 | 17 July 2012 |
| | | US | 8308644 | B2 | 13 November 2012 |
| | | US | 8354239 | B2 | 15 January 2013 |
| | | US | 8435181 | B2 | 07 May 2013 |
| | | US | 8802450 | B2 | 12 August 2014 |
| | | US | 9383353 | B2 | 05 July 2016 |
| | | US | 9993225 | B2 | 12 June 2018 |
| | | WO | 2004-012584 | A2 | 12 February 2004 |
| | | WO | 2004-012584 | A3 | 06 May 2004 |
| | | WO | 2004-017834 | A1 | 04 March 2004 |
| | | WO | 2004-041094 | A2 | 21 May 2004 |
| | | WO | 2004-041094 | A3 | 21 May 2004 |
| | | WO | 2005-000390 | A2 | 06 January 2005 |
| | | WO | 2005-000390 | A3 | 31 March 2005 |
| | | WO | 2005-044109 | A1 | 19 May 2005 |
| | | WO | 2005-079487 | A2 | 01 September 2005 |
| | | WO | 2005-079487 | A3 | 13 November 2008 |
| | | WO | 2005-107581 | A2 | 17 November 2005 |
| | | WO | 2005-107581 | A3 | 08 March 2007 |
| | | WO | 2005-107581 | A8 | 20 September 2007 |
| | | WO | 2005-112773 | A2 | 01 December 2005 |
| | | WO | 2005-112773 | A3 | 21 June 2007 |
| | | WO | 2006-017168 | A2 | 16 February 2006 |
| | | WO | 2006-017168 | A3 | 04 May 2006 |
| | | WO | 2006-026605 | A2 | 09 March 2006 |
| | | WO | 2006-026605 | A3 | 22 February 2007 |
| | | WO | 2006-031526 | A2 | 23 March 2006 |
| | | WO | 2006-031526 | A3 | 15 March 2007 |
| | | WO | 2006-047554 | A2 | 04 May 2006 |
| | | WO | 2006-047554 | A3 | 20 July 2006 |
| | | WO | 2006-062867 | A2 | 15 June 2006 |
| | | WO | 2006-062867 | A3 | 09 August 2007 |
| | | WO | 2007-016369 | A2 | 08 February 2007 |
| | | WO | 2007-016369 | A3 | 23 April 2009 |
| | | WO | 2007-103737 | A2 | 13 September 2007 |
| | | WO | 2007-103737 | A3 | 19 June 2008 |
| | | WO | 2008-073560 | A2 | 19 June 2008 |
| | | WO | 2008-073560 | A3 | 04 December 2008 |
| | | WO | 2008-083386 | A2 | 10 July 2008 |
| | | WO | 2008-083386 | A3 | 28 August 2008 |
| | | WO | 2008-144449 | A2 | 27 November 2008 |
| | | WO | 2008-144449 | A3 | 30 December 2009 |
| | | WO | 2008-144452 | A1 | 27 November 2008 |
| | | WO | 2008-144570 | A1 | 27 November 2008 |
| | | WO | 2010-017508 | A1 | 11 February 2010 |
| JP 2003-190168 A | 08 July 2003 | None | | | |

Form PCT/ISA/210 (patent family annex) (July 2019)

**EP 4 454 569 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100763453
- KR 101874613